(19) Europäisches Patentamt / European Patent Office / Office européen des brevets

(11) **EP 2 959 906 A1**

(12) **EUROPEAN PATENT APPLICATION**
published in accordance with Art. 153(4) EPC

(43) Date of publication:
**30.12.2015 Bulletin 2015/53**

(21) Application number: **14754624.6**

(22) Date of filing: **24.02.2014**

(51) Int Cl.:
*A61K 33/44* (2006.01)    *A61P 1/16* (2006.01)
*A61P 13/12* (2006.01)    *A61P 39/02* (2006.01)
*B01J 20/20* (2006.01)    *C01B 31/10* (2006.01)

(86) International application number:
**PCT/JP2014/054261**

(87) International publication number:
**WO 2014/129614 (28.08.2014 Gazette 2014/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.02.2013   JP 2013033615**

(71) Applicant: **Kureha Corporation**
**Chuo-ku**
**Tokyo 103-8552 (JP)**

(72) Inventors:
• **SONOBE, Naohiro**
**Tokyo 103-8552 (JP)**
• **WAKAHOI, Takashi**
**Tokyo 103-8552 (JP)**
• **AKITA, Takahiro**
**Tokyo 103-8552 (JP)**

(74) Representative: **Zimmermann & Partner**
**Patentanwälte mbB**
**Postfach 330 920**
**80069 München (DE)**

(54) **ORALLY ADMINISTERED ADSORBENT, THERAPEUTIC AGENT FOR RENAL DISEASE, AND THERAPEUTIC AGENT FOR LIVER DISEASE**

(57) An object of the present invention is to provide an orally administered adsorbent capable of adsorbing large quantities of tryptophan or indoxyl sulfate in the presence of bile acid. The problem can be solved by an orally administered adsorbent comprising spherical active carbon having bulk density from 0.30 to 0.46 g/mL, specific surface area determined by the Brunauer-Emmett-Teller (BET) method of not less than 2000 $m^2$/g, total acid group content of less than 0.30 meq/g, pore volume of pore diameter from 20 to 10,000 nm of not more than 0.21 mL/g; and a micropore volume ratio (Vm) of not less than 1.0, determined by Formula (1): Vm = $(V_{2.0}-V_{1.1})/(V_{1.1}-V_{0.64})$ (1) [in Formula (1), $V_{2.0}$, $V_{1.1}$, and $V_{0.64}$ are cumulative pore volumes of pore diameters not greater than 2.0 nm, not greater than 1.1 nm, and not greater than 0.64 nm, respectively, calculated by the SF method from nitrogen adsorbed quantity].

EP 2 959 906 A1

**Description**

TECHNICAL FIELD

The present invention relates to an orally administered adsorbent comprising spherical activated carbon having low bulk density, high specific surface area, total acidic group content of less than 0.30 meq/g, pore volume of pore diameter from 20 nm to 10,000 nm of not greater than 0.21 mL/g, and a high proportion of pore volume of pore diameter not less than 1.1 nm among micropores (pores not greater than 2 nm). The present invention also relates to a therapeutic or prophylactic agent for a renal disease and a therapeutic or prophylactic agent for a hepatic disease that contain as an active ingredient the aforementioned orally administered adsorbent. The orally administered adsorbent according to the present invention has high adsorption ability for indoxyl sulfate and its precursor tryptophan, which are poisonous toxic substances (toxin) in the body, in the presence of a high concentration of bile acid, and therefore can adsorb many toxic substances in the residence period in the body from oral ingestion to discharge to outside the body.

BACKGROUND

Accompanying organ functional impairment in patients deficient in renal function or hepatic function, poisonous toxic substances accumulate and are produced in the body such as in the blood, and cause uremia or encephalopathy such as impaired consciousness. Since the number of such patients has shown an increasing trend year by year, the development of therapeutic medicines or organ substitute devices that has the function of removing toxic substances to outside the body in place of these deficient organs is a critical topic. Removal of poisonous substances by hemodialysis is currently the method most widely used as artificial kidneys. However, such hemodialysis-type artificial kidneys are not necessarily satisfactory due to problems such as the necessity for a specialized technician from the viewpoint of safety management because a special machine is used, and additionally, the high physical, mental and economic burden on the patient due to extracorporeal removal of blood, and the like.

As a means for solving these problems, an oral adsorbent that can be orally ingested and can treat functional impairment of the kidney or liver has been developed and used (Patent Document 1). The oral adsorbent has been widely clinically used in, for example, patients with hepatorenal functional impairment as an oral therapeutic agent that has less side effect such as constipation. The oral adsorbent contains a porous spherical carbonaceous substance (that is, spherical activated carbon) having a certain functional group, has excellent adsorbance of poisonous substances (that is, β-aminoisobutyric acid, γ-amino-n-butyric acid, dimethylamine, and octopamine) in the presence of bile acid in the intestines, and also has beneficial selective adsorbance in the sense that it adsorbs little of the beneficial components in the intestines such as digestive enzymes and the like. Furthermore, the adsorbent described in Patent Document 1 uses pitch such as petroleum pitch as a carbon source, and is produced by performing oxidation treatment and reduction treatment after preparation of spherical activated carbon.

On the other hand, it is known that in chronic renal failure patients, serum indoxyl sulfate concentration sometimes increases to approximately 60 times that of normal people, and it is also known that serum indoxyl sulfate concentration is reduced and the progression of renal failure is slowed by administration of the oral adsorbent described in Patent Document 1 (Non-Patent Documents 1 and 2).

CITATION LIST

Patent Literature

Patent Document 1: Japanese Examined Patent Application Publication No. S62-11611B
Patent Document 2: Japanese Unexamined Patent Application Publication No. 2006-131461A
Patent Document 3: Japanese Unexamined Patent Application Publication No. 2006-15334A
Patent Document 4: Japanese Unexamined Patent Application Publication No. 2011-37749A

Non-Patent Literature

Non-Patent Document 1: Japanese Journal of Nephrology, Vol. 32, No. 6 (1990) pp. 65-71
Non-Patent Document 2: Japanese Journal of Clinical Dialysis, Vol. 14, No. 4 (1998), pp. 433-438

SUMMARY OF INVENTION

Technical Problem

Adsorption of toxic substances is an extremely important characteristic of an oral adsorbent containing spherical activated carbon, but it is particularly important that indoxyl sulfate and its precursor tryptophan, which are toxic substances in chronic renal failure patients, are adsorbed and removed in large quantity and as quickly as possible in the intestinal environment. That is, various substances are present in large quantities, and a particularly large quantity of bile acid (15 mM) is present in the human intestines. Therefore, spherical activated carbon having excellent adsorption ability for toxic substances in the small intestine in which a large quantity of bile acid is present is desired.
An object of the present invention is to provide an orally administered adsorbent capable of adsorbing large quantities of tryptophan or indoxyl sulfate in the presence of bile acid.
As a result of diligent research on oral adsorbents capable of adsorbing and removing a large quantity of toxic substances in the presence of a high concentration of bile acid, the present inventors discovered that an oral adsorbent exhibiting excellent adsorption ability even in the presence of bile acid is obtained using spherical activated carbon having low bulk density, high specific surface area, total acidic group content of less than 0.30 meq/g, pore volume of pore diameter from 20 nm to 10,000 nm of not greater than 0.21 mL/g, and a high proportion of pore volume of pore diameter not less than 1.1 nm among micropores (pores not greater than 2 nm). The above spherical activated carbon discovered by the present inventors is capable of adsorbing large quantities of toxic substances (particularly indoxyl sulfate and its precursor tryptophan) even in the presence of a high concentration of bile acid, and enables reduced dosage. The present invention is based on such knowledge.

Solution To Problem

Therefore, the present invention relates to the following.

[1] An orally administered adsorbent containing spherical activated carbon having bulk density from 0.30 g/mL to 0.46 g/mL, specific surface area determined by the Brunauer-Emmett-Teller (BET) method of not less than 2000 $m^2$/g, total acidic group content of less than 0.30 meq/g, pore volume of pore diameter from 20 nm to 10,000 nm of not greater than 0.21 mL/g, and a micropore volume ratio (Vm) determined by Formula (1) of not less than 1.0:
Formula (1)

$$Vm = (V_{2.0}-V_{1.1})/(V_{1.1}-V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the Saito-Foley (SF) method from nitrogen adsorbed quantity);
[2] The orally administered adsorbent according to [1], wherein the average particle size of the spherical activated carbon is from 50 to 200 $\mu$m;
[3] The orally administered adsorbent according to [1] or [2], wherein the spherical activated carbon is prepared using crosslinked vinyl resin as a carbon source;
[4] A therapeutic or prophylactic agent for a renal disease containing as an active ingredient the orally administered adsorbent described in any one of [1] to [3]; and
[5] A therapeutic or prophylactic agent for a hepatic disease containing as an active ingredient the orally administered adsorbent described in any one of [1] to [3]. Furthermore, the present specification discloses the following.
[6] A method of prophylaxis or therapy of a renal disease or a hepatic disease wherein the orally administered adsorbent described in any one of [1] to [3] is administered in an effective dose to a renal disease or hepatic disease therapy subject;
[7] Spherical activated carbon for use in a therapy (method) of a renal disease or a hepatic disease, the spherical activated carbon having bulk density from 0.30 g/mL to 0.46 g/mL, specific surface area determined by the BET method of not less than 2000 $m^2$/g, total acidic group content of less than 0.30 meq/g, pore volume of pore diameter from 20 nm to 10,000 nm of not greater than 0.21 mL/g, and a micropore volume ratio (Vm) determined by Formula (1) of not less than 1.0:
Formula (1)

$$Vm = (V_{2.0}-V_{1.1})/(V_{1.1}-V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the SF method from nitrogen adsorbed quantity];

[8] The spherical activated carbon according to [7], wherein the average particle size of the spherical activated carbon is from 50 $\mu$m to 200 $\mu$m;

[9] The spherical activated carbon according to [7] or [8], wherein the spherical activated carbon is prepared using crosslinked vinyl resin as a carbon source;

[10] Use of spherical activated carbon for production of a prophylactic or therapeutic medicine for a renal disease or a hepatic disease, the spherical activated carbon having bulk density from 0.30 g/mL to 0.46 g/mL, a specific surface area determined by the BET method of not less than 2000 $m^2$/g, total acidic group content of less than 0.30 meq/g, pore volume of the pore diameter from 20 nm to 10,000 nm of not greater than 0.21 mL/g, and a micropore volume ratio (Vm) determined by Formula (1) of not less than 1.0:

Formula (1)

$$Vm = (V_{2.0}-V_{1.1})/(V_{1.1}-V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the SF method from nitrogen adsorbed quantity];

[11] The use of spherical activated carbon according to [10], wherein the average particle size of the spherical activated carbon is from 50 $\mu$m to 200 $\mu$m;

[12] The use of spherical activated carbon according to [10] or [11], wherein the spherical activated carbon is prepared using crosslinked vinyl resin as a carbon source;

[13] Use of spherical activated carbon for prophylaxis or therapy of a renal disease or a hepatic disease, the spherical activated carbon having bulk density from 0.30 g/mL to 0.46 g/mL, a specific surface area determined by the BET method of not less than 2000 $m^2$/g, total acidic group content of less than 0.30 meq/g, pore volume of pore diameter from 20 nm to 10,000 nm of not greater than 0.21 mL/g, and a micropore volume ratio (Vm) determined by Formula (1) of not less than 1.0:

Formula (1)

$$Vm = (V_{2.0}-V_{1.1})/(V_{1.1}-V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the SF method from nitrogen adsorbed quantity];

[14] The use of spherical activated carbon according to [13], wherein the average particle size of the spherical activated carbon is from 50 $\mu$m to 200 $\mu$m;

[15] The use of spherical activated carbon according to [13] or [14], wherein the spherical activated carbon is prepared using crosslinked vinyl resin as a carbon source.

Advantageous Effects of Invention

The orally administered adsorbent according to the present invention can very rapidly adsorb poisonous toxic substances in the intestines because adsorption ability for toxic substances in the presence of bile acid is high due to the use of spherical activated carbon having low bulk density, a high specific surface area, low total acidic group content, pore volume of pore diameter from 20 nm to 10,000 nm of not greater than 0.21 mL/g, and a high proportion of pore volume of pore diameter not less than 1.1 nm among micropores (pores not greater than 2 nm). Therefore, the orally administered adsorbent according to the present invention is effective as a therapeutic or prophylactic agent of a renal disease or as a therapeutic or prophylactic agent of a hepatic disease. Additionally, the dosage can be reduced to the dosage less than the dosage of a conventional orally administered adsorbent.

In particular, because bulk density is low, the number of particles per unit weight is large and the adsorbent disperses widely, and as a result, poisonous toxic substances can be extremely rapidly adsorbed because the migration length of toxic substances to the orally administered adsorbent is short. Additionally, due to the fact that the number of particles per unit weight is increased, the exterior surface area of the particles per unit weight through which the toxic substances must pass to be adsorbed is increased, and as a result, poisonous toxic substances can be extremely rapidly adsorbed.

Brief Description of Drawings

FIG. 1 illustrates the results of a potassium indoxyl sulfate adsorption test in the presence of bile acid of the spherical activated carbon obtained in Working Examples 1 to 3 and Comparative Example 1.
FIG. 2 illustrates the results of a tryptophan adsorption test in the presence of bile acid of the spherical activated carbon obtained in Working Examples 1 to 3 and Comparative Example 1.

Description of Embodiments

[1] Orally administered adsorbent

The spherical activated carbon used for the orally administered adsorbent according to the present invention, as described above, has bulk density, specific surface area, total acidic group content, pore volume of pore diameter from 20 nm to 10,000 nm, and micropore volume ratio within certain ranges. Specifically, the spherical activated carbon has bulk density from 0.30 g/mL to 0.46 g/mL, a specific surface area determined by the BET method of not less than 2000 $m^2$/g, total acid group content of less than 0.30 meq/g, pore volume of pore diameter from 20 nm to 10,000 nm of not greater than 0.21 mL/g, and a micropore volume ratio (Vm) determined by Formula (1) of not less than 1.0:

$$Vm = (V_{2.0}\text{-}V_{1.1})/(V_{1.1}\text{-}V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the SF method from nitrogen adsorbed quantity].

(Bulk density)

The bulk density of the spherical activated carbon used in the present invention is from 0.30 g/mL to 0.46 g/mL.
The upper limit of bulk density is preferably less than 0.44 g/mL, more preferably less than 0.42 g/mL, and most preferably not greater than 0.40 g/mL. This is because spherical activated carbon having low bulk density has excellent adsorption ability for indoxyl sulfate and its precursor tryptophan in the presence of bile acid. The lower limit of bulk density is 0.30 g/mL, more preferably 0.31 g/mL, and most preferably 0.32 g/mL. This is because spherical activated carbon having low bulk density has excellent adsorption ability for toxic substances, but, on the other hand, as bulk density decreases, the yield of spherical activated carbon becomes worse, resulting in a decrease in economy in the production of the activated carbon. Additionally, when bulk density is too low, the spherical activated carbon is readily crushed and does not maintain a spherical shape because the strength of spherical activated carbon decreases. Furthermore, in this specification, bulk density $\rho_B$ is the value obtained by dividing the dry weight W (g) of the spherical activated carbon when the spherical activated carbon is packed in a container by the volume V (mL) of the packed spherical activated carbon, and can be obtained by the following calculation formula.

[Formula 1]

$$\rho_{B(g/mL)} = \frac{W_{(g)}}{V_{(mL)}}$$

The bulk density of spherical activated carbon is a good index for indicating the degree of activation. Specifically, the lower the bulk density, the more activation has proceeded. In the manufacturing process of spherical activated carbon, in steam activation to be described later, relatively small pores are formed in the early phase of activation, and the pore diameter increases as activation proceeds, resulting in bulk density decreasing.

(Specific surface area)

The specific surface area of the spherical activated carbon can be determined by the BET method or the Langmuir method. The spherical activated carbon used for the orally administered adsorbent according to the present invention has a specific surface area determined by the BET method (sometimes abbreviated as "SSA" hereinafter) of not less than 2000 $m^2$/g. When spherical activated carbon has SSA of less than 2000 $m^2$/g, toxin adsorption performance in the presence of bile acid decreases, which is undesirable. The upper limit of SSA is not particularly limited, but from the perspective of bulk density and strength, SSA is preferably not greater than 3000 $m^2$/g.

(Total acidic groups)

The spherical activated carbon used for the orally administered adsorbent according to the present invention is surface-unmodified spherical activated carbon. Here, surface-unmodified spherical activated carbon means spherical activated carbon having total acidic groups of less than 0.30 meq/g. Conversely, surface-modified spherical activated carbon means spherical activated carbon having total acidic groups of not less than 0.30 meq/g. As will be described later, surface-unmodified spherical activated carbon is a porous body obtained by performing activation treatment after heat-treating a carbon precursor, for example. It may be spherical activated carbon which has not subsequently undergone surface modification treatment by oxidation treatment and reduction treatment, or it may be spherical activated carbon obtained by performing heat treatment in a non-oxidative atmosphere after the aforementioned activation treatment. On the other hand, surface-modified spherical activated carbon is a porous body obtained by performing activation treatment after heat-treating a carbon precursor, and then further performing surface modification treatment by oxidation treatment and reduction treatment. It may exhibit appropriate degrees of interaction with acids and bases.
The spherical activated carbon used in the orally administered adsorbent of the present invention is surface-unmodified spherical activated carbon, and therefore, the total acidic groups is less than 0.30 meq/g, preferably not greater than 0.25 meq/g, and more preferably not greater than 0.20 meq/g.

(Pore volume)

The pore volume of pore diameter from 20 nm to 10,000 nm of the spherical activated carbon used in the orally administered adsorbent of the present invention is not greater than 0.21 mL/g, preferably not greater than 0.20 mL/g, and more preferably not greater than 0.19 mL/g. When the pore volume of pore diameter from 20 nm to 10,000 nm exceeds 0.21 mL/g, it is undesirable because the adsorbed quantity of useful substances such as digestive enzymes may increase. The lower limit is not particularly limited, but is preferably not less than 0.04 mL/g.
The pore volume of pore diameter from 20 nm to 10,000 nm is measured using the mercury penetration method.
The pore volume of pore diameter from 7.5 nm to 15,000 nm of the spherical activated carbon used in the orally administered adsorbent of the present invention is not less than 0.01 mL/g, preferably not less than 0.1 mL/g, more preferably not less than 0.2 mL/g, even more preferably not less than 0.4 mL/g, and most preferably not less than 0.5 mL/g. By having a large pore volume of pore diameter from 7.5 nm to 15,000 nm, the adsorption rate of poisonous substance is excellent. The upper limit of pore volume of pore diameter from 7.5 nm to 15,000 nm is not particularly limited, but is preferably not greater than 1.0 mL/g. When the pore volume of pore diameter from 7.5 nm to 15,000 nm exceeds 1.0 mL/g, it is undesirable because the adsorbed quantity of useful substances such as digestive enzymes may increase.

(Micropore volume ratio)

According to the International Union of Pure and Applied Chemistry (IUPAC), pores not greater than 2 nm are defined as micropores, those from 2 nm to 50 nm are defined as mesopores, and those not less than 50 nm are defined as macropores. The spherical activated carbon used for the orally administered adsorbent according to the present invention mainly forms relatively small micropores through gas activation. Due to these micropores being formed, the density of the spherical activated carbon is reduced and the specific surface area is increased, thereby increasing toxin adsorption performance in the presence of bile acid.
The pore volume of micropores not greater than 2 nm can be measured by the nitrogen adsorption method, and can be analyzed by the Saito-Foley method (called "SF method" hereinafter), the Horvath-Kawazoe method, the density functional theory method, and the like, but in the present invention, pore volume obtained by the SF method, which assumes that pore shape is cylindrical, is used.

Specifically, in the spherical activated carbon used in the orally administered adsorbent of the present invention, the micropore volume ratio (Vm) determined by Formula (1) is not less than 1.0, preferably not less than 1.1, and more preferably not less than 1.2:

$$Vm = (V_{2.0} - V_{1.1})/(V_{1.1} - V_{0.64}) \qquad (1)$$

(wherein, in the formula, $V_{2.0}$, $V_{1.1}$, and $V_{0.64}$ are the cumulative pore volumes of pore diameter not greater than 2.0 nm, not greater than 1.1 nm, and not greater than 0.64 nm, respectively, calculated by the SF method from nitrogen adsorbed quantity]. That is, in the spherical activated carbon used in the orally administered adsorbent of the present invention, the proportion of pore volume of pore diameter from 1.1 nm to 2 nm is high among micropores, which are pores not greater than 2 nm. When the micropore volume ratio is not greater than 1.0, it is undesirable because the relatively large bile acid molecules end up causing pore blockage, and adsorption of uremic substances and their precursors of relatively small molecular size ends up being inhibited. When the micropore volume ratio increases, the relatively large bile acid molecules do not cause pore blockage, and adsorption of uremic substances and their precursors of relatively small molecular size is excellent. Therefore, the upper limit is not particularly limited, but not greater than 1.5 is preferred.

(Diameter)

The diameter of the spherical activated carbon used for the orally administered adsorbent according to the present invention is not particularly limited, but is preferably 0.01 mm to 1 mm, and more preferably from 0.02 mm to 0.8 mm. When the diameter of the spherical activated carbon is less than 0.01 mm, the exterior surface area of the spherical activated carbon increases and adsorption of beneficial substances such as digestive enzymes readily occurs, which is undesirable. When the diameter exceeds 1 mm, the diffusion length of toxic substances into the spherical activated carbon increases and the adsorption rate decreases, which is undesirable.

(Average particle size)

In this specification, an average particle size means the particle diameter at a cumulative particle size percentage of 50% on a volume standard cumulative particle size distribution curve (Dv50).
The range of average particle size of the spherical activated carbon used for the orally administered adsorbent according to the present invention is not particularly limited, but is preferably from 0.01 mm to 1 mm. When the average particle size of the spherical activated carbon is less than 0.01 mm, the exterior surface area of the spherical activated carbon increases and adsorption of beneficial substances such as digestive enzymes readily occurs, which is undesirable. When the average particle size exceeds 1 mm, the diffusion length of toxic substances into the spherical activated carbon increases and the adsorption rate decreases, which is undesirable. The average particle size is preferably from 0.02 mm to 0.8 mm, because spherical activated carbon having an average particle size particularly from 50 μm to 200 μm has excellent initial adsorption ability, and in the general residence period in the upper small intestine, can very rapidly adsorb poisonous toxic substances in the body. A more preferred range of average particle size is from 50 μm to 170 μm, and an even more preferred range is from 50 μm to 150 μm.

(Particle size distribution)

The spherical activated carbon used for the orally administered adsorbent according to the present invention preferably has a narrow particle size distribution. For example, when expressed by the ratio ($D_4/D_1$) of the weight average particle size $D_4$ of the weight-based distribution ($= \sum(nD^4)/\sum(nD^3)$) to the average particle size $D_1$ ($= \sum nD/\sum n$) of the number-based average length, the spherical activated carbon used for the orally administered adsorbent according to the present invention preferably has a ratio ($D_4/D_1$) of not greater than 3, more preferably not greater than 2, and even more preferably not greater than 1.5. Here, the closer the aforementioned ratio ($D_4/D_1$) is to 1, the narrower the particle size distribution. Furthermore, in the above calculation formula, D is the typical particle size of measured particle size classification, and n is the number of particles.

(Effect)

In the past it has been completely unknown that spherical activated carbon having the above bulk density, specific surface area, total acidic group content, pore volume, and micropore volume ratio has high adsorption ability for toxic substances (particularly indoxyl sulfate and its precursor tryptophan, which are toxic substances in chronic renal failure patients) in the presence of bile acid. For example, Patent Document 1 describes a carbonaceous adsorbent capable of adsorbing β-aminoisobutyric acid, γ-amino-n-butyric acid, dimethylamine, and octopamine in a 0.5 wt% bile salt aqueous solution. However, the carbonaceous adsorbents of Working Examples 1 to 3 in Patent Document 1 were activated for 2 hours at 900°C. Activation under such temperature and time conditions is thought to result in a specific surface area of less than 2000 $m^2$/g, and the spherical activated carbon described in Patent Document 1 differs in specific surface area from the spherical activated carbon of the present invention.

Additionally, because the spherical activated carbon described in Patent Document 1 is surface-modified spherical activated carbon which has undergone oxidation and reduction, the value of its total acidic group content differs from that of the spherical activated carbon of the present invention.

Furthermore, Patent Document 2 generally describes activated carbon having a packing density of not less than 0.3 g/mL (for example, claim 1). Also, the working examples and comparative examples describe activated carbon having bulk density (packing density) from 0.306 g/mL to 0.460 g/mL. For these activated carbons, the BET specific surface area is not stated and it is unclear whether or not the BET specific surface area is not less than 2000 $m^2$/g, and it is unclear whether or not pore volume of pore diameter from 20 nm to 10,000 nm is not greater than 0.21 mL/g, but it is clear that the micropore volume ratio is not greater than 1.0, and the physical properties differ completely from those of the spherical activated carbon used for the orally administered adsorbent of the present invention. Patent Document 2 in no way describes that an orally administered adsorbent containing spherical activated carbon having low bulk density, a high specific surface area, a micropore volume ratio of not less than 1.0, and pore volume of pore diameter from 20 nm to 10,000 nm of not greater than 0.21 mL has high adsorption ability for toxic substances in the presence of bile acid.

Additionally, Patent Document 3 describes activated carbon having a packing density from 0.345 g/mL to 0.455 g/mL in working examples (Working Examples 1, 3, 5, 9, 10, 13, and the like). For this activated carbon, the total acidic group content and micropore volume ratio are unclear, but the specific surface area and pore volume of pore diameter from 20 nm to 10,000 nm are completely different from those of the spherical activated carbon used for the orally administered adsorbent of the present invention. Additionally, Patent Document 4 describes activated carbons having packing densities of 0.42 g/mL and 0.44 g/mL in Working Examples 1 and 2. For these activated carbons, the micropore volume ratio is unclear, but the specific surface area and total acidic group content are completely different from those of the spherical activated carbon used for the orally administered adsorbent of the present invention. Moreover, Patent Documents 3 and 4 in no way describe that an orally administered adsorbent containing spherical activated carbon having low bulk density, a high specific surface area, a micropore volume ratio of not less than 1.0, and pore volume of pore diameter from 20 nm to 10,000 nm of not greater than 0.21 mL has high adsorption ability for toxic substances in the presence of bile acid.

The reason that the orally administered adsorbent according to the present invention has such excellent effects is unclear at present, but can be hypothesized as follows. However, the present invention is not limited to the following hypothesis.

A high concentration of bile acid is present in the body. Bile acid is a type of surfactant for aiding absorption of lipids that are not readily soluble in water. Therefore, in order for bile acid to micellate lipids, a concentration higher than the critical micelle concentration is required, and bile acid is present in a concentration of 15 mM in the human small intestine when satiated. Typical examples of bile acid include sodium cholate, sodium deoxycholate, sodium taurocholate, sodium glycocholate, and the like, which are relatively large molecules having a molecular weight from approximately 400 to 600. Additionally, when bile acid molecules meet and micellate, they form a large micelle size of several nm, which is larger than indoxyl sulfate and its precursor tryptophan, which have gained attention as poisonous toxic substances (toxin) in the body. The spherical activated carbon of the present invention is thought to have an excellent effect because, due to having the specified bulk density and specific surface area, it has high adsorption ability for uremic toxins and their precursors, and due to having a high micropore volume ratio, large bile acid molecules and bile acid micelles do not cause hindrance to adsorption such as pore blockage, and it can adsorb indoxyl sulfate and its precursor tryptophan, which are small uremic toxins. Furthermore, due to having a low pore volume of pore diameter from 20 nm to 10,000 nm, the spherical activated carbon of the present invention can prevent adsorption of beneficial substances such as digestive enzymes.

(Carbon source)

The spherical activated carbon used for the orally administered adsorbent of the present invention may use any carbon-containing material as a carbon source. Examples of carbon-containing materials that can be used include synthetic resin and pitch. As the synthetic resin, heat-fusible resin or heat-infusible resin may be used. Here, heat-fusible resin is resin that ends up melting or decomposing as the temperature rises when activation treatment is performed without infusibility treatment, and that cannot yield activated carbon. However, when activation treatment is performed after infusibility treatment has been performed, the heat-fusible resin can be used as activated carbon. In contrast, heat-infusible resin carbonizes as the temperature rises even when activation treatment is performed without infusibility treatment, and can yield activated carbon. Furthermore, infusibility treatment means, for example, oxidation treatment at a temperature from 150°C to 400°C in an atmosphere containing oxygen, as will be described later.

Atypical example of heat-fusible resin is thermoplastic resin, for example, crosslinked vinyl resin. On the other hand, a typical example of heat-infusible resin is thermosetting resin, examples of which include phenol resin and furan resin. Among known thermoplastic resins and thermosetting resins, any that can form spheres may be used. Furthermore, the above-described infusibility treatment is required when obtaining spherical activated carbon from crosslinked vinyl resin, whereas it is unnecessary when obtaining spherical activated carbon from ion exchange resin produced by adding a functional group to crosslinked vinyl resin. This is because crosslinked vinyl resin is considered to have been modified from heat-fusible resin to heat-infusible resin by the introduced functional group or functional group addition treatment. That is, crosslinked vinyl resin is included among heat-fusible resins in this specification, whereas ion exchange resin is included among heat-infusible resins in this specification.

The carbon source of the spherical activated carbon used in the present invention is not particularly limited, but the use of a synthetic resin is preferred due to its ease of handling. Examples of synthetic resins include thermosetting resins (for example, phenol resin and furan resin) and ion exchange resins, which are heat-infusible resins, and thermoplastic resins (for example, crosslinked vinyl resin), which are heat-fusible resins. Here, with thermosetting resins, voids are readily formed in the spherical activated carbon and strength decreases, and when crushed, there is the danger of it piercing the intestines. With ion exchange resin, attention is required when used in oral administration because the ion exchange resin contains sulfur components, and the like. Accordingly, use of a thermoplastic resin (for example, crosslinked vinyl resin) as the carbon source of spherical activated carbon is more preferred.

(Heat-fusible resin)

When heat-fusible resin (for example, crosslinked vinyl resin) is used as a carbon source, substantially the same operations as conventional production methods using pitch can be employed. The above spheres formed of heat-fusible resin soften and deform into a non-spherical shape or fuse to each other when heat-treated. Therefore, softening can be reduced by performing oxidation treatment at a temperature from 150°C to 400°C using an oxidant as infusibility treatment prior to activation treatment. $O_2$ or a mixed gas of oxygen diluted with air, nitrogen, or the like may be used as the oxidant.

Crosslinked vinyl resin, which is a heat-fusible resin, softens and melts by heat treatment in a non-oxidative gas atmosphere and has a carbonization yield of less than 10%, but the crosslinked vinyl resin does not soften and melt by oxidation treatment in an atmosphere containing oxygen at a temperature from 150°C to 400°C as infusibility treatment, and a spherical carbonaceous material can be obtained at a high carbonization yield of not less than 30%, and by performing activation treatment on this spherical carbonaceous material, spherical activated carbon can be obtained. When a large amount of pyrolysis gas is generated in heat-treatment of the spheres of the heat-fusible resin after infusibility treatment, pyrolysis products can be removed by appropriate pre-heating before performing the activation operation.

Then, spherical activated carbon can be obtained by activation treatment under the gas flow that reacts with carbon (for example, steam or carbon dioxide) at a temperature from 700°C to 1000°C. In this specification, "activated carbon" means a porous body obtained by performing activation treatment after heat-treating a carbon precursor such as spherical heat-fusible resin, and "spherical activated carbon" means activated carbon having a spherical shape and a specific surface area of not less than 100 m$^2$/g. The average particle size of the spheres of heat-fusible resin used as the starting material is not particularly limited, but approximately from 0.02 mm to 1.5 mm is preferred, from 50 μm to 800 μm is more preferred, and from 70 μm to 500 μm is even more preferred.

The above crosslinked vinyl resin used as a starting material may be a spherical polymer obtained by emulsion polymerization, bulk polymerization, or solution polymerization, or, preferably, a spherical polymer obtained by suspension polymerization. To make the spherical crosslinked vinyl resin having a diameter of not less than 50 μm uniformly infusible, it is necessary to form pores in the crosslinked vinyl resin in advance. Pores may be formed in the resin by adding a porogen at the time of polymerization. The BET specific surface area of the crosslinked vinyl resin required to make the crosslinked vinyl resin infusible is preferably not less than 10 m$^2$/g, and more preferably not less than 50 m$^2$/g. For example, when preparing a crosslinked vinyl resin by suspension polymerization, a spherical crosslinked vinyl resin may be prepared by adding an organic phase containing a vinyl-based monomer, a crosslinking agent, a porogen, and a polymerization initiator to an aqueous dispersion medium containing a dispersion stabilizer, and after forming numerous organic droplets suspended in an aqueous phase by agitating to mix, and heating them to polymerize the monomer in the organic droplets.

As vinyl-based monomers, any vinyl-based monomer that can be formed into spheres can be used, examples of which include aromatic vinyl-based monomers such as styrene and styrene derivatives in which a vinyl group hydrogen or phenyl group hydrogen is substituted, and compounds in which a heterocyclic or polycyclic compound is bonded to a vinyl group instead of a phenyl group. More specific examples of aromatic vinyl-based monomers include α- or β-methylstyrene, α- or β-ethylstyrene, methoxystyrene, phenylstyrene, chlorostyrene, and the like, and o-, m-, or p-methylstyrene, ethylstyrene, methoxystyrene, methylsilylstyrene, hydroxystyrene, chlorostyrene, cyanostyrene, nitrostyrene, aminostyrene, and carboxystyrene, and sulfoxystyrene, sodium styrene sulfonate, and the like, and vinylpyridine, vinylthiophene, vinylpyrrolidone, vinylnaphthalene, vinylanthracene, vinylbiphenyl, and the like. Aliphatic vinyl-based monomers may also be used, specific examples of which include ethylene, propylene, isobutylene, diisobutylene, vinyl chloride, acrylic acid ester, methacrylic acid ester, vinyl esters such as vinyl acetate, and the like, vinyl ketones such as vinyl methyl ketone, vinyl ethyl ketone, and the like, vinyl aldehydes such as acrolein, methacrolein, and the like, vinyl ethers such as vinyl methyl ether, vinyl ethyl ether, and the like, and vinyl nitriles such as acrylonitrile, ethylacrylonitrile, diphenylacrylonitrile, chloroacrylonitrile, and the like.

As the crosslinking agent, any crosslinking agent that can be used to crosslink the above vinyl-based monomers may be used, examples of which include divinylbenzene, divinylpyridine, divinyltoluene, divinylnaphthalene, diallyl phthalate, ethylene glycol diacrylate, ethylene glycol dimethylate, divinylxylene, divinylethylbenzene, divinylsulfone, and polyvinyl or polyallyl ethers of glycol or glycerol, polyvinyl or polyallyl ethers of pentaerythritol, polyvinyl or polyallyl ethers of mono or dithio derivatives of glycol, and polyvinyl or polyallyl ethers of resorcinol, and divinyl ketone, divinyl sulfide, allyl acrylate, diallyl maleate, diallyl fumarate, diallyl succinate, diallyl carbonate, diallyl malonate, diallyl oxalate, diallyl adipate, diallyl sebacate, triallyl tricarballylate, triallyl aconitate, triallyl citrate, triallyl phosphate, N,N'-methylenediacrylamide, 1,2-di(α-methylmethylenesulfonamide)ethylene, trivinylbenzene, trivinylnaphthalene, polyvinylanthracene, and trivinylcyclohexane. Particularly preferred crosslinking agents include polyvinyl aromatic hydrocarbons (for example, divinylbenzene), glycol trimethacrylates (for example, ethylene glycol dimethacrylate), and polyvinyl hydrocarbons (for example, trivinylcyclohexane). Divinylbenzene is most preferred due to its excellent pyrolysis characteristics.

Examples of suitable porogens include alkanols having from 4 to 10 carbons (for example, n-butanol, sec-butanol, 2-ethylhexanol, decanol, and 4-methyl-2-pentanol), alkyl esters having at least 7 carbons (for example, n-hexyl acetate, 2-ethylhexyl acetate, methyl oleate, dibutyl sebacate, dibutyl adipate, and dibutyl carbonate), alkyl ketones having from 4 to 10 carbons (for example, dibutyl ketone and methyl isopropyl ketone), alkyl carboxylates (for example, heptanoic acid), aromatic hydrocarbons (for example, toluene, xylene, and benzene), higher saturated aliphatic hydrocarbons (for example, hexane, heptane, and isooctane), and cyclic aliphatic hydrocarbons (for example, cyclohexane).

The polymerization initiator is not particularly limited, and one that is generally used in this field may be used, but an oil-soluble polymerization initiator that is soluble in the polymerizable monomer is preferred. Examples of the polymerization initiator include dialkyl peroxides, diacyl peroxides, peroxyesters, peroxydicarbonates, and azo compounds. More specific examples include dialkyl peroxides such as methyl ethyl peroxide, di-t-butyl peroxide, and dicumyl peroxide; diacyl peroxides such as isobutyl peroxide, benzoyl peroxide, 2,4-dicyclobenzoyl peroxide, and 3,5,5-trimethylhexanoyl peroxide; peroxyesters such as t-butyl peroxypivalate, t-hexyl peroxypivalate, t-butyl peroxyneodecanoate, t-hexyl peroxyneodecanoate, 1-cyclohexyl-1-methylethyl peroxyneodecanoate, 1,1,3,3-tetramethylbutyl peroxyneodecanoate, cumyl peroxyneodecanoate, and (α,α-bis-neodecanoylperoxy)diisopropylbenzene; peroxydicarbonates such as bis(4-t-butylcyclohexyl)peroxydicarbonate, di-n-propyl-oxydicarbonate, diisopropyl peroxydicarbonate, di(2-ethylethylperoxy)dicarbonate, dimethoxybutyl peroxydicarbonate, and di(3-methyl-3-methoxybutylperoxy)dicarbonate; and azo compounds such as 2,2'-azobisisobutyronitrile, 2,2'-azobis(4-methoxy-2,4-dimethylvaleronitrile), 2,2'-azobis(2,4-dimethylvaleronitrile), and 1,1'-azobis(1-cyclohexanecarbonitrile); and the like.

(Heat-infusible resin)

When heat-infusible resin (for example, thermosetting resin or ion exchange resin) is used as a carbon source in the preparation of the spherical activated carbon used for the orally administered adsorbent of the present invention, substantially the same operations as conventional production methods using pitch can be employed. For example, spherical activated carbon can be obtained by first performing activation treatment under the gas flow that reacts with carbon (for example, steam or carbon dioxide gas) at a temperature from 700°C to 1000°C on spheres formed of heat-infusible resin. Furthermore, similar to the case of the above heat-fusible resin, when a large amount of pyrolysis gas is generated in heat-treatment of the spheres of the heat-infusible resin, pyrolysis products can be removed in advance by appropriate pre-heating before performing the activation operation.

The average particle size of the spheres of heat-infusible resin used as the starting material is not particularly limited, but approximately from 0.02 mm to 1.5 mm is preferred, from 50 μm to 800 μm is more preferred, and from 70 μm to 500 μm is even more preferred.

The above heat-infusible resin used as a starting material is a material that can be formed into spheres and, importantly, does not melt or soften and undergo shape deformation in heat treatment at a temperature not greater than 500°C.

For the above heat-infusible resin used as the starting material, it is desirable that carbonization yield by heat treatment is high. When carbonization yield is low, strength as spherical activated carbon will be low. Since unnecessary pores are otherwise formed, the bulk density of the spherical activated carbon decreases and the specific surface area per unit volume decreases, and therefore the administered volume increases, which leads to the problem that oral administration is difficult. Therefore, a higher carbonization yield of the heat-infusible resin is preferred, and the value of yield by heat treatment in a non-oxidizing gas atmosphere at 800°C is preferably not less than 30 wt%, and more preferably not less than 35 wt%.

Specific examples of the thermosetting resin used as the starting material include phenol resins such as novolac-type phenol resins, resole-type phenol resins, novolac-type alkylphenol resins, and resole-type alkylphenol resins, and furan resins, urea resins, melamine resins, epoxy resins, and the like may also be used. Copolymers of divinylbenzene with styrene, acrylonitrile, acrylic acid, or methacrylic acid may also be used as the thermosetting resin.

Furthermore, ion exchange resin may be used as the heat-infusible resin. The ion exchange resin is generally formed of a copolymer of divinylbenzene with styrene, acrylonitrile, acrylic acid, or methacrylic acid (that is, a crosslinked vinyl resin which is a heat-fusible resin), and basically has a structure in which an ion exchange group is bonded to a copolymer matrix having a three-dimensional mesh skeleton. Depending on the ion exchange group, ion exchange resins are broadly classified into strongly acidic ion exchange resins having a sulfonic acid group, weakly acidic ion exchange resins having a carboxylic acid group or sulfonic acid group, strongly basic ion exchange resins having a quaternary ammonium salt, and weakly basic ion exchange resins having a primary or tertiary amine. Other special resins include so-called hybrid ion exchange resins having ion exchange groups of both an acid and a base. In the present invention, all of these ion exchange resins may be used as a raw material.

When activation treatment is performed under the gas flow that reacts with carbon (for example, steam or carbon dioxide) at a temperature from 700°C to 1000°C using heat-infusible resin as a carbon source, spherical activated carbon having a ratio of pore volume of pore diameter from 1.1 nm to 2 nm relative to pore volume of pore diameter from 0.64 nm to 1.1 nm determined by the SF method using the nitrogen adsorption method of not less than 1.0 can be obtained.

(Control of physical properties in synthetic resin)

When preparing the spherical activated carbon according to the present invention using the above heat-fusible resin or heat-infusible resin, the physical properties of the spherical activated carbon (for example, average particle size, pore volume, particle size distribution, specific surface area, and the like) can be controlled by various methods. For example, the average particle size and particle size distribution of resin depends on the size of the droplets in the aqueous phase, and the size of the droplets can be controlled by the amount of suspending agent, the rotational frequency of stirring, the shape of the stirring blades, or the monomer ratio in the aqueous phase (ratio of amount of water and amount of monomer). For example, when the amount of suspending agent is increased, droplet size decreases, and when the rotational frequency of stirring is increased, droplet size decreases. Additionally, it is preferred that the amount of monomer in the aqueous phase is decreased from the perspective that not only coalescence of droplets can be controlled, but removal of heat of polymerization becomes easy as well. However, when the monomer ratio is too low, the amount of monomer per batch becomes small and the amount of synthetic resin obtained decreases, which is undesirable from the perspective of productivity.

Furthermore, when the pore diameter to be controlled is not less than 10 nm, the pore volume and specific surface area can be controlled primarily by the amount and type of porogen, and when the pore diameter to be controlled is not greater than 10 nm, the pore volume and specific surface area can be controlled by activation conditions using steam. Additionally, the fine structure as spherical activated carbon can be controlled by the type of resin, the type and amount of crosslinking agent, the infusibility conditions, the heating conditions, and/or the activation temperature, and the like.

As the activation reaction, spherical activated carbon used for the orally administered adsorbent of the present invention can be obtained by activation treatment under the gas flow that reacts with carbon (for example, steam or carbon dioxide) at a temperature from 700°C to 1000°C. The bulk density can be controlled by the activation conditions. For example, the bulk density can be decreased by increasing the activation time, increasing the activation temperature, and increasing the concentration of the gas flow that reacts with carbon.

(Pitch)

When pitch is used as the carbon source in the preparation of the spherical activated carbon used for the orally administered adsorbent of the present invention, it may be prepared by, for example, the following method.

A bicyclic or tricyclic aromatic compound or mixture thereof having a boiling point of not less than 200°C is added as an additive to pitch such as petroleum pitch or coal pitch, and after mixing while heating, the mixture is formed to produce pitch formed bodies. The size of the pitch formed bodies can be controlled by the nozzle diameter used in extrusion molding or the crushing conditions of the pitch formed bodies. The smaller the volume of the pitch formed bodies, the smaller the spherical pitch that can be produced, and the smaller the particle size of spherical activated carbon that can be obtained.

Next, the pitch formed bodies are dispersed and granulated while stirring in hot water at 50°C to 120°C, and after they become tiny spheres, they are cooled, and spherical pitch formed bodies are obtained. The average particle size of the spherical pitch formed bodies is not particularly limited, but approximately from 0.02 mm to 1.5 mm is preferred, from 60 $\mu$m to 350 $\mu$m is more preferred, and from 60 $\mu$m to 300 $\mu$m is even more preferred. Additionally, in a solvent having low solubility for pitch and high solubility for the additive, the additive is extracted from the spherical pitch formed bodies, and the obtained porous pitch is made into infusible porous pitch by oxidation using an oxidant. Spherical activated carbon can be obtained by treatment of the obtained heat-infusible porous pitch under the gas flow that reacts with carbon, for example, steam or carbon dioxide, at a temperature from 800°C to 1000°C.

In particular, to produce microspheres of spherical activated carbon having an average particle size of approximately 50 $\mu$m to 200 $\mu$m, it is preferable to perform control such as increasing the temperature when spinning the pitch and naphthalene, increasing the amount of polyvinyl alcohol, or cooling in a short time.

The purpose of the aromatic additive described above is to make it easy to extract the additive from the pitch formed bodies after forming to make the formed bodies porous and to make it easy to control the structure of and heat of the carbonaceous material by oxidation in the subsequent step. Such an additive is selected from one or a mixture of two or more aromatic compounds such as, for example, naphthalene, methylnaphthalene, phenylnaphthalene, benzylphenylnaphthalene, methylanthracene, phenanthrene, and biphenyl. The added amount relative to the pitch is preferably in the range from 10 to 50 parts by weight relative to 100 parts by weight of pitch.

To achieve a homogeneous mixture of the pitch and the additive, they are mixed in the molten state while heating. Forming may be performed in the molten state, or by cooling the mixture and then crushing, but a method of extruding the mixed pitch into a thread in the molten state and then cutting it at even intervals or crushing it is preferred because particle size distribution can be controlled in a narrower range. Particle size can be controlled by the nozzle diameter when extruding the mixed pitch, and smaller mixture formed bodies can be obtained by using a narrower nozzle. As the solvent used for extracting the additive from the mixture of pitch and additive, an aliphatic hydrocarbon such as butane, pentane, hexane, or heptane, or a mixture of aliphatic hydrocarbon main constituents such as naphtha or kerosene, or an aliphatic alcohol such as methanol, ethanol, propanol, or butanol, or the like is preferred.

By extracting the additive from the formed bodies of the mixture of pitch and additive using such a solvent, the additive can be removed from the formed bodies while the spherical shape of the formed bodies is maintained. It is surmised that holes are formed by the additive in the formed bodies at this time, and pitch formed bodies having uniform porosity can be obtained.

The porous pitch formed bodies obtained in this manner are then made into heat-infusible porous infusible pitch formed bodies by infusibility treatment, namely oxidation treatment preferably at a temperature from 150°C to 400°C using an oxidant. $O_2$ or a mixed gas of oxygen diluted with air, nitrogen, or the like may be used as the oxidant.

When pitch is used as the carbon source in the preparation of the spherical activated carbon used for the orally administered adsorbent of the present invention, pore volume can be controlled by controlling the amount and type of aromatic additive and the precipitation conditions within the pitch, and spherical activated carbon having pore volume of pore diameter from 20 nm to 10,000 nm of not greater than 0.21 mL/g can be prepared.

For the above pitch used as the starting material, it is desirable that carbonization yield by heat treatment is high. When carbonization yield is low, strength as spherical activated carbon will be low. Since unnecessary pores are otherwise formed, the bulk density of the spherical activated carbon decreases and the specific surface area per unit volume decreases, and therefore the administered volume increases, which leads to the problem that oral administration is difficult. Therefore, a higher carbonization yield of the pitch is preferred, and yield by heat treatment in a non-oxidizing gas atmosphere at 800°C is preferably not less than 50 wt%, and more preferably not less than 60 wt%.

(Surface modification)

Surface-modified spherical activated carbon can be obtained by performing oxidation treatment on the spherical activated carbon obtained with heat-fusible resin, heat-infusible resin, or pitch as a carbon source in an atmosphere containing from 0.1 vol% to 50 vol% oxygen, preferably from 1 vol% to 30 vol%, and particularly preferably from 3 vol% to 20 vol% oxygen, at a temperature from 300°C to 800°C, and preferably from 320°C to 600°C, and then performing reduction treatment at a temperature from 800°C to 1200°C, and preferably from 800°C to 1000°C, in a non-oxidative gas atmosphere. Here, surface-modified spherical activated carbon is a porous body obtained by performing oxidation treatment and reduction treatment on the above spherical activated carbon.

However, the spherical activated carbon used for the orally administered adsorbent of the present invention may be used as-is in that state without performing subsequent steps of an oxidation step and a reduction step for supporting a functional group.

(Physical properties of spherical activated carbon)

The physical property values, namely average particle size, bulk density, specific surface area, pore volume, and particle size distribution, of the spherical activated carbon used for the orally ingested adsorbent according to the present invention are measured by the following methods.

(1) Average particle size (Dv50)

The particle diameter at a cumulative particle size percentage of 50% on a volume standard cumulative particle size distribution curve created using a laser diffraction-style particle size distribution analyzer (SALAD-3000S; Shimadzu Corp.) was used as the average particle size (Dv50).

(2) Bulk density

Measurement was performed in accordance with the packing density measurement method of JIS K 1474-5.7.2.

(3) Specific surface area (specific surface area calculation method by BET method)

The gas adsorption quantity of a spherical activated carbon sample can be measured using a specific surface area analyzer that uses the gas adsorption method (for example, ASAP2010 or ASAP2020; Micromeritics Corp.), and a specific surface area can be calculated using the formula below. Specifically, the spherical activated carbon sample is put into a sample tube, and after vacuum-drying at 350°C, post-drying sample weight is measured. Then, the sample tube is cooled to -196°C and nitrogen is introduced into the sample tube to adsorb nitrogen on the spherical activated carbon sample, and the relationship between nitrogen partial pressure and adsorbed quantity (adsorption isotherm) is measured.
A BET plot is created, with the relative pressure of nitrogen taken as p and the adsorbed quantity at that time taken as v (cm$^3$/g STP). Specifically, the range of p from 0.05 to 0.20 is plotted with p/ (v (1-p)) on the vertical axis and p on the horizontal axis, and the specific surface area S (units: m$^2$/g) is determined by the following formula from the slope b (units: g/cm$^3$) and intercept c (units: g/cm$^3$) at that time.

[Formula 2]

$$S = \frac{MA \times \left(6.02 \times 10^{23}\right)}{22414 \times 10^{18} \times (b + c)}$$

MA is the cross-sectional area of a nitrogen molecule, and a value of 0.162 nm$^2$ was used here.

(4) Specific surface area (specific surface area calculation method by Langmuir equation)

The gas adsorption quantity of a spherical activated carbon sample can be measured using a specific surface area analyzer that uses the gas adsorption method (for example, ASAP2010 or ASAP2020; Micromeritics Corp.), and a specific surface area can be calculated using the Langmuir equation. Specifically, the spherical activated carbon sample is put into a sample tube, and after vacuum-drying at 350°C, post-drying sample weight is measured. Then, the sample tube is cooled to -196°C and nitrogen is introduced into the sample tube to adsorb nitrogen on the spherical activated carbon sample, and the relationship between nitrogen partial pressure and adsorbed quantity (adsorption isotherm) is measured.
A Langmuir plot is created, with the relative pressure of nitrogen taken as p and the adsorbed quantity at that time taken as v (cm$^3$/g STP). Specifically, the range of p from 0.05 to 0.20 is plotted with p/v on the vertical axis and p on the horizontal axis, and the specific surface area S (units: m$^2$/g) is determined by the following formula when the slope at that time is taken as b (units: g/cm$^3$).

[Formula 3]

$$S = \frac{MA \times \left(6.02 \times 10^{23}\right)}{22414 \times 10^{18} \times b}$$

MA is the cross-sectional area of a nitrogen molecule, and a value of 0.162 nm$^2$ was used here.

(5) Pore distribution (Saito-Foley calculation formula)

The relationship between nitrogen partial pressure and adsorbed quantity (adsorption isotherm) of a spherical activated carbon sample was measured at liquid nitrogen temperature (-196°C) using a specific surface area analyzer that uses the gas adsorption method (ASAP2010 or ASAP2020; Micromeritics Corp.). From the obtained adsorption isotherm, pore distribution was calculated by the Saito-Foley calculation formula (Saito, A. and Foley, H.C., Ache Journal 37 (3), 429 (1991)) using analysis software that comes with the aforementioned specific surface area analyzer (ASAP2010 or ASAP2020; Micromeritics Corp.). Pore shape analyzed by slit geometry was that obtained by the original Horvath-Kawazoe calculation method (Horvath, G. and Kawazoe, K., J. Chem. Eng. Japan 16 (6), 470 (1983)), but since the structure of the carbon is a three-dimensionally disarrayed structure of non-graphitizable carbon, calculation by cylinder geometry (Saito, A. and Foley, H.C., AlChE Journal 37 (3), 429 (1991)) was chosen.
The various parameters used in the calculation are as follows.

Interaction parameter: 1.56 $\times$ 10$^{-43}$ ergs·cm$^4$
Diameter of adsorptive molecule: 0.3000 nm
Diameter of sample molecule: 0.3400 nm
Density conversion factor: 0.001547 (cm$^3$ liquid/cm$^3$ STP)

(6) Pore volume by mercury penetration method

Pore volume can be measured using a mercury porosimeter (for example, Autopore 9200; Micromeritics Corp.). The spherical activated carbon sample is put in a sample container, and degassed for 30 minutes under pressure not greater than 2.67 Pa. Then, mercury is introduced into the sample container, pressure is gradually increased, and the mercury penetrates into the pores of the spherical activated carbon sample (maximum pressure: 414 MPa). From the relationship between pressure and mercury penetration quantity at this time, the pore volume distribution of the spherical activated carbon sample is measured using the calculation formulas below.
Specifically, the volume of mercury that penetrates the spherical activated carbon sample is measured from a pressure equivalent to pore diameter 21 $\mu$m (0.06 MPa) to the maximum pressure (414 MPa, equivalent to pore diameter 3 nm). In the calculation of pore diameter, when mercury penetrates into the pores of a cylinder having a diameter (D) at a pressure (P), and the surface tension of mercury is taken as "$\gamma$" and the contact angle between mercury and the pore wall is taken as "$\theta$," the following equation

$$-\pi D \gamma \cos\theta = \pi (D/2)^2 \cdot P$$

holds true based on the equilibrium of surface tension and the pressure that acts on the pore cross-section. Therefore,

$$D = (-4\gamma\cos\theta)/P$$

In this specification, the surface tension of mercury is taken as 484 dyne/cm and the contact angle between mercury and carbon is taken as 130 degrees. When the pressure P is expressed in MPa and the pore diameter D is expressed in $\mu$m, the relationship between the pressure P and the pore diameter D is determined using the following formula:

$$D = 1.24/P$$

For example, the pore volume in the range of pore diameter from 20 nm to 10,000 nm is equivalent to the volume of mercury that penetrates at mercury penetration pressure from 0.124 MPa to 62 MPa. Also, the pore volume in the range of pore diameter from 7.5 nm to 15,000 nm is equivalent to the volume of mercury that penetrates at mercury penetration pressure from 0.083 MPa to 165 MPa.

Furthermore, because the spherical activated carbon used for the orally administered adsorbent of the present invention has an extremely small particle size, the spaces between sample particles packed in the sample container are also small. Therefore, in the operation of pore volume measurement by the above mercury penetration method, there is a stage at which the mercury penetrates those interparticle spaces, and at that penetration stage, it behaves as if there are pores having a pore diameter from 8000 nm to 15,000 nm. It can be confirmed by observation using, for example, an electron microscope that no pores having a pore diameter from 8000 nm to 15,000 nm are present in the spherical activated carbon used for the orally administered adsorbent of the present invention. Therefore, in this specification, "pore volume in the range of pore diameter from 20 nm to 15,000 nm" and "pore volume in the range of pore diameter from 7.5 to 15,000 nm" also include the amount of mercury that penetrates the interparticle spaces.

(7) Particle size distribution

The number particle size distribution was measured using a laser diffraction-type particle size distribution analyzer (SALAD-3000S; Shimadzu Corp.). The typical particle size D of measured particle size classification and the number of particles n in that measured particle size classification were determined, and the length average particle size $D_1$ and the weight average particle size $D_4$ were calculated by the following formula.

[Formula 4]

$$D_1 = \frac{\sum (nD)}{\sum n}$$

[Formula 5]

$$D_4 = \frac{\sum (nD^4)}{\sum (nD^3)}$$

(8) Total acidic group content

1 g of spherical activated carbon sample was added to 50 mL of 0.05 N NaOH solution, and this was shaken in a figure-eight with an amplitude of 3 cm at 76 cycles/minute at 37°C for 48 hours using a figure-eight shaker (Triple Shaker NR-80; Taitec Corp.). The spherical activated carbon sample was then filtered out, and the consumed amount of NaOH determined by neutralization titration was taken as the total acidic group content.

The orally administered adsorbent of the present invention contains the above-described spherical activated carbon as an active ingredient, but it may contain only spherical activated carbon or may also contain pharmaceutically acceptable additives other than spherical activated carbon. Examples of additives include excipients, disintegration agents, surfactants, binders, lubricants, acidulants, foaming agents, sweeteners, fragrances, colorants, stabilizers, and flavoring agents.

When the orally administered adsorbent is formed of spherical activated carbon, the administered form may be, for example, a powder, granules, capsule, or individual package. When the orally administered adsorbent contains spherical activated carbon and additives, the administered form may be, for example, a powder, granules, tablet, sugar-coated pill, capsule, suspension, stick, individual package, or emulsion.

[2] Orally administered adsorbent for therapy or prophylaxis of renal disease or hepatic disease

Because the spherical activated carbon used for the orally administered adsorbent of the present invention has excellent adsorbance of hepatic disease aggravating factors and toxic substances in renal diseases, the spherical activated carbon may be used as an orally administered adsorbent for therapy or prophylaxis of a renal disease or may be used as an orally administered adsorbent for therapy or prophylaxis of a hepatic disease. Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is predialysis mild renal failure, and it may be used for condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition).
Examples of the hepatic disease include fulminant hepatitis, chronic hepatitis, viral hepatitis, alcoholic hepatitis, hepatic fibrosis, cirrhosis, hepatic cancer, autoimmune hepatitis, drug-induced allergic hapatic disorder, primary biliary cirrhosis, tremor, encephalopathy, metabolic disorder, and functional disorder. Otherwise, it may also be used in therapy of illnesses caused by toxic substances present in the body, that is, mental illness and the like.

Therefore, when the orally administered adsorbent according to the present invention is used as a therapeutic medicine for a renal disease, the orally administered adsorbent contains the above spherical activated carbon as an active ingredient. When the orally administered adsorbent of the present invention is used as a therapeutic medicine for a renal disease or a therapeutic medicine for a hepatic disease, the dosage thereof is influenced by whether the subject of administration is a human or other animal, and by age, individual differences, disease condition, or the like. Therefore, depending on the case, a dosage outside the following range may be appropriate, but in general, the orally administered dosage in humans is from 1 g to 20 g per day divided into three to four doses, and may be further adjusted according to symptoms. The administered form may be a powder, granules, tablet, sugar-coated pill, capsule, suspension, stick, individual package, emulsion, or the like. When ingested as a capsule, in addition to an ordinary gelatin capsule, an enteric-coated capsule may be used as necessary. When used as a tablet, it needs to disintegrate into microparticles in the body. Additionally, it may be used in the form of a complex blended with an electrolyte regulator such as alumigel or Kayexalate, which are other preparations.

Spherical activated carbon having bulk density from 0.30 g/mL to 0.46 g/mL, a specific surface area determined by the BET method of not less than 2000 $m^2$/g, total acidic group content of less than 0.30 meq/g, pore volume of pore diameter from 20 nm to 10,000 nm of not greater than 0.21 mL/g, and a micropore volume ratio (Vm) of not less than 1.0 can be used as a therapeutic or prophylactic agent for a renal disease or a therapeutic or prophylactic agent for a hepatic disease in the form of a mixture with a conventional known surface-modified spherical activated carbon or spherical activated carbon (that is, spherical activated carbon having bulk density exceeding 0.46 g/mL, specific surface area of less than 2000 $m^2$/g, total acidic group content of not less than 0.30 meq/g, pore volume of pore diameter from 20 nm to 10,000 nm exceeding 0.21 mL/g, and a micropore volume ratio (Vm) of less than 1.0, or having a combination of those physical properties).
Alternatively, spherical activated carbon having bulk density from 0.30 g/mL to 0.46 g/mL, a specific surface area determined by the BET method of not less than 2000 $m^2$/g, total acidic group content of less than 0.30 meq/g, pore volume of pore diameter from 20 nm to 10,000 nm of not greater than 0.21 mL/g, and a micropore volume ratio (Vm) of not less than 1.0 and a conventional known surface-modified spherical activated carbon or spherical activated carbon (that is, spherical activated carbon having bulk density exceeding 0.46 g/mL, a specific surface area of less than 2000 $m^2$/g, total acidic group content of not less than 0.30 meq/g, pore volume of pore diameter from 20 nm to 10,000 nm exceeding 0.21 mL/g, and a micropore volume ratio (Vm) of less than 1.0, or having a combination of those physical properties) can be used concomitantly as a therapeutic or prophylactic agent for a renal disease or a therapeutic or prophylactic agent for a hepatic disease.

[3] Method of therapy of renal disease or hepatic disease

The spherical activated carbon used in the orally administered adsorbent according to the present invention can be used in a method of prophylaxis or therapy of a renal disease or a hepatic disease. Therefore, the method of therapy of a renal disease or a hepatic disease of the present invention is characterized in that the above orally administered adsorbent containing spherical activated carbon is administered in an effective dose to a renal disease or hepatic disease therapy subject.
The administration route, dosage, administration interval, and the like of the above spherical activated carbon may be determined as appropriate in accordance with the type of illness, the age, gender, and body weight of the patient, the degree of symptoms, the dosing method, and the like.

[4] Spherical activated carbon for use in method of therapy of renal disease or hepatic disease

The spherical activated carbon used in the orally administered adsorbent according to the present invention can be used in a method of prophylaxis or therapy of a renal disease or a hepatic disease. Therefore, the spherical activated carbon of the present invention is for use in a method of prophylaxis or therapy of a renal disease or a hepatic disease.
The amount and the like of the above spherical activated carbon used in prophylaxis or therapy may be determined as appropriate in accordance with the type of illness, the age, gender, and body weight of the patient, the degree of symptoms, the dosing method, and the like.

[5] Use of spherical activated carbon for production of therapeutic medicine for renal disease or hepatic disease

The spherical activated carbon used in the orally administered adsorbent according to the present invention can be used for producing a prophylactic or therapeutic medicine for a renal disease or a hepatic disease. Therefore, use of the present invention is use of spherical activated carbon for producing a medicine for prophylaxis or therapy of a renal disease or a hepatic disease.
The contained amount and the like of the above spherical activated carbon in the prophylactic or therapeutic medicine may be determined as appropriate in accordance with the type of illness, the age, gender, and body weight of the patient, the degree of symptoms, the dosing method, and the like.

[6] Use of spherical activated carbon for therapy of renal disease or hepatic disease

The spherical activated carbon used in the orally administered adsorbent according to the present invention can be used for therapy of a renal disease or a hepatic disease. Therefore, use of the present invention is use of spherical activated carbon for prophylaxis or therapy of a renal disease or a hepatic disease.
The amount and the like of the above spherical activated carbon used in prophylaxis or therapy may be determined as appropriate in accordance with the type of illness, the age, gender, and body weight of the patient, the degree of symptoms, the dosing method, and the like.

[Examples]

The present invention will be described in detail hereinafter using working examples, but these working examples do not limit the scope of the present invention.

Working Example 1

4800 g of deionized water, 7.2 g of methylcellulose, and 1.0 g of sodium nitrite were put in a 10-L polymerization can. To this were added 481 g of styrene, 1119 g of 57% pure divinylbenzene (57% divinylbenzene and 43% ethylvinylbenzene), 9.3 g of 2,2'-azobis(2,4-dimethylvaleronitrile), and 560 g of hexane as a porogen, and the interior of the system was then replaced with nitrogen gas. This two-phase system was heated to 55°C while stirring at 140 rpm, and then held in that state for 20 hours. The obtained resin was filtered, and then hexane was removed from the resin by evaporation by vacuum-drying. The resulting resin was vacuum-dried for 12 hours at 90°C, to produce spherical porous synthetic resin having an average particle size of 246 $\mu$m. The specific surface area of the porous synthetic resin was approximately 240 m$^2$/g.
The obtained spherical porous synthetic resin was put in a reactor with a grating, and infusibility treatment was performed in a vertical tube furnace. As the infusibility conditions, dry air was made to flow from bottom to top of the reaction tube, and after heating to 190°C, the temperature was raised from 190°C to 290°C at a rate of 10°C/h, and spherical porous oxidized resin was thereby obtained. The spherical porous oxidized resin was heated in a nitrogen atmosphere at 850°C, and then, using a fluidized bed, activation treatment was performed in a nitrogen gas atmosphere containing steam at 850°C until the bulk density reached 0.46 g/mL, and spherical activated carbon was thereby obtained.

Working Example 2

Spherical activated carbon was obtained by repeating the operations of Working Example 1 except that instead of performing activation treatment until the bulk density reached 0.46 g/mL, activation treatment was performed until the bulk density reached 0.40 g/mL.

Working Example 3

Spherical activated carbon was obtained by repeating the operations of Working Example 1 except that instead of performing activation treatment until the bulk density reached 0.46 g/mL, activation treatment was performed until the bulk density reached 0.30 g/mL.

Working Example 4

Spherical phenol resin (trade name "Industrial Phenol Resin Resitop (Maririn HF-100, product no. 60303)"; Gunei Chemical Industry Co., Ltd.) was put in a quartz vertical reaction tube with a grating, heated to 300°C in 0.5 hours under nitrogen gas flow, then heated to 700°C in 2 hours, and then held for 30 minutes. After that, activation treatment was performed in a nitrogen gas atmosphere containing steam at 850°C until the bulk density reached 0.40 g/mL, and spherical activated carbon was thereby obtained.

Working Example 5

695 g of petroleum-based pitch having a softening point of 210°C, quinoline-insoluble components of not greater than 1 wt%, and H/C atom ratio of 0.63, and 305 g of naphthalene were put in a 3-L pressure-resistant container equipped with stirring blades, and after being melted and mixed at 180°C, the mixture was cooled to 155°C and extruded through a 0.75-mm nozzle to produce a string-like formed body. Then, the string-like formed body was crushed and then fractionated using sieves with openings from 150 $\mu$m to 212 $\mu$m. The obtained crushed matter was put in an aqueous solution in which 0.46 wt% polyvinyl alcohol (degree of saponification 88%) was dissolved, and made into spheres by stirring to disperse for 50 minutes at 90°C. The dispersion was then cooled to 40°C in 3 minutes, and the pitch was solidified and naphthalene crystallized, to produce a spherical pitch formed body slurry. After the majority of the water was removed by filtration, the naphthalene in the pitch formed bodies was extracted with n-hexane in a quantity of 6 times the weight of the spherical pitch formed bodies. Using a fluidized bed, the porous spherical pitch obtained in this manner was heated to 240°C and held for 1 hour at 240°C while hot air was passed through to oxidize, thereby producing heat-infusible porous spherical oxidized pitch. The porous spherical oxidized pitch was heated in a nitrogen atmosphere at 850°C, and then, using a fluidized bed, activation treatment was performed in a nitrogen gas atmosphere containing steam at 850°C until the bulk density reached 0.38 g/mL, and spherical activated carbon was thereby obtained.

Comparative Example 1

Spherical activated carbon was obtained by repeating the operations of Working Example 1 except that instead of performing activation treatment until the bulk density reached 0.46 g/mL, activation treatment was performed until the bulk density reached 0.50 g/mL.

Comparative Example 2

The operations of Working Example 1 were repeated, except that instead of performing activation treatment until the bulk density reached 0.46 g/mL, activation treatment was performed until the bulk density reached 0.25 g/mL. However, it ended up being crushed because strength was poor, and spherical activated carbon could not be obtained.

Comparative Example 3

The operations of Working Example 5 were repeated except that instead of cooling to 155°C after the petroleum pitch and naphthalene were melted and mixed at 180°C, the mixture was cooled to 140°C, and instead of the crushed matter of a string-like formed body being put in an aqueous solution in which 0.46 wt% polyvinyl alcohol was dissolved, the crushed matter was put in an aqueous solution in which 0.23 wt% polyvinyl alcohol was dissolved, and instead of it being cooled to 40°C in 90 minutes after being stirred to disperse for 50 minutes at 95°C to make spheres, it was cooled to 40°C in 3 minutes. However, it ended up being crushed because strength was poor, and spherical activated carbon could not be obtained.

Comparative Example 4

Using a fluidized bed, the spherical activated carbon obtained in Comparative Example 1 underwent oxidation treatment for 3 hours at 470°C in air.

Comparative Example 5

Spherical phenol resin (trade name "Industrial Phenol Resin Resitop (Maririn HF-100, product no. 60303)"; Gunei Chemical Industry Co., Ltd.) was put in a 500°C quartz vertical reaction tube with a grating, held for 30 minutes under nitrogen gas flow, then heated to 700°C in 20 minutes, and then held at 700°C for 1 hour. After that, activation treatment was performed at 900°C until the bulk density reached 0.46 g/mL, and spherical activated carbon was thereby obtained.

(Method for evaluating oral adsorbent)

The various characteristics shown in Tables 1 and 2 below were measured by the following methods.

(1) Average particle size

Average particle size was measured using the above-described laser diffraction-style particle size distribution analyzer.

(2) Pore volume

The micropore volume of each of the spherical activated carbons obtained in the working examples and comparative examples was determined by the SF method using the nitrogen adsorption method, and the pore volume of pore diameter from 20 nm to 10,000 nm and the pore volume of pore diameter from 7.5 nm to 15,000 nm were determined by the above-described mercury penetration method.

(3) Specific surface area by BET method and Langmuir method

Specific surface area was measured by the above BET method and the Langmuir method.

(4) Bulk density

A 50-mL graduated cylinder was filled with the sample to the 50-mL mark, and after tapping 50 times, the sample weight was divided by the volume to determine bulk density. Results are shown in Tables 1 and 2. The measured values obtained by this method and the measured values obtained by the packing density measurement method of JIS K 1474-5.7.2 did not differ at all within the range of significant digits shown in Tables 1 and 2.

(5) Total acidic group content

1 g of spherical activated carbon sample was added to a 50 mL of 0.05 N NaOH solution, and this was shaken in a figure-eight with an amplitude of 3 cm at 76 cycles/minute at 37°C for 48 hours using a figure-eight shaker (Triple Shaker NR-80; Taitec Corp.). The spherical activated carbon sample was then filtered out, and the consumed amount of NaOH determined by neutralization titration was taken as the total acidic group content.

(6) Potassium indoxyl sulfate adsorption test

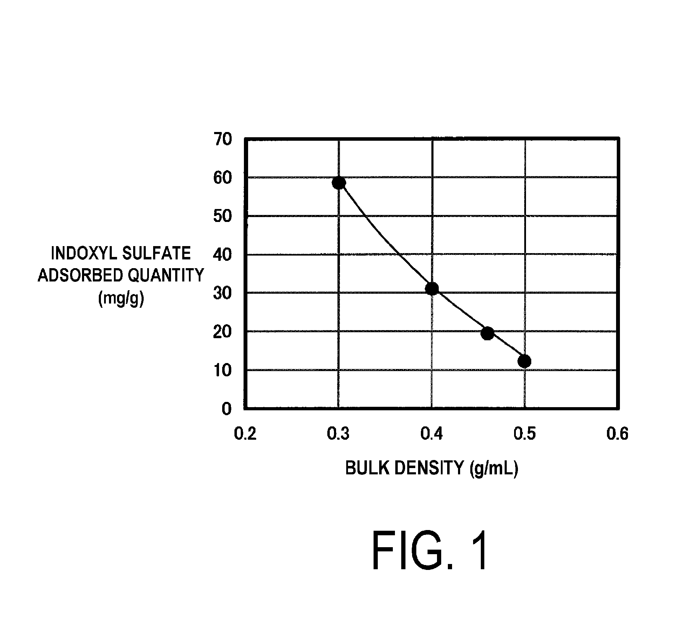

After the spherical activated carbon sample was dried, 0.05 g of dry sample was weighed out and put in a 50-mL screw-cap sample bottle. Meanwhile, 100 mg of potassium indoxyl sulfate and 6458 g of sodium cholate were precisely weighed out, and phosphate buffer solution of pH 7.4 was added and dissolved to make precisely 1000 mL (potassium indoxyl sulfate stock solution), and precisely 50 mL of this stock solution was added to the aforementioned 50-mL screw-cap sample bottle. The resulting solution was shaken for 2 hours at $37 \pm 1°C$ at 10 rpm using a mix rotor (Mix Rotor Variable VMR-5R; Asone Corp.). The contents in the screw-cap sample bottle were suction-filtered using a membrane filter with 0.65-$\mu$m pores, and approximately 20 mL of the first filtrate was removed. Approximately 10 mL of the next filtrate was diluted with acetonitrile, and a sample solution with a ratio of filtrate:acetonitrile = 1:1 was obtained. To make calibration curve solutions, 0 mL, 25 mL, 50 mL, 75 mL, and 100 mL of the potassium indoxyl sulfate stock solution were precisely measured and transferred into measuring flasks, and calibration curve stock solutions were prepared by adding phosphate buffer solution of pH 7.4 to make a total of 100 mL. These solutions were then diluted with acetonitrile, and calibration curve solutions with a ratio of calibration curve stock solution:acetonitrile = 1:1 were obtained.
Absorbance at wavelength 278 nm of the sample solution and calibration curve solutions was measured using high-performance liquid chromatography (HPLC), and the potassium indoxyl sulfate adsorbed quantity (mg/g) was calculated. The results are shown in Table 2. Furthermore, the results of Working Examples 1 to 3 and Comparative Example 1 are shown in FIG. 1. As is clear from FIG. 1, as bulk density of spherical activated carbon decreases, potassium indoxyl sulfate adsorbed quantity increases dramatically in the presence of bile acid.

(7) Tryptophan adsorption test

A tryptophan adsorption test was performed by the following method for the various spherical activated carbons and surface-modified spherical activated carbons obtained in Working Examples 1 to 5 and Comparative Examples 1, 4, and 5.

After the spherical activated carbon sample was dried, 0.01 g of dry sample was weighed out and put in a 50-mL screw-cap sample bottle. Meanwhile, 100 mg of tryptophan and 6458 g of sodium cholate were precisely weighed out, and phosphate buffer solution of pH 7.4 was added and dissolved to make precisely 1000 mL (tryptophan stock solution), and precisely 50 mL of this stock solution was added to the aforementioned 50-mL screw-cap sample bottle. The resulting solution was shaken for 2 hours at $37\pm1°C$ at 10 rpm using a mix rotor (Mix Rotor Variable VMR-5R; Asone Corp.). The contents in the screw-cap sample bottle were suction-filtered using a membrane filter with 0.65-$\mu$m pores, and approximately 20 mL of the first filtrate was removed. Approximately 10 mL of the next filtrate was diluted with acetonitrile, and a sample solution with a ratio of filtrate:acetonitrile = 1:1 was obtained.

To make calibration curve solutions, 0 mL, 25 mL, 50 mL, 75 mL, and 100 mL of the tryptophan stock solution were precisely measured and transferred into measuring flasks, and calibration curve stock solutions were prepared by adding phosphate buffer solution of pH 7.4 to make a total of 100 mL. These solutions were then diluted with acetonitrile, and calibration curve solutions with a ratio of calibration curve stock solution:acetonitrile = 1:1 were obtained.

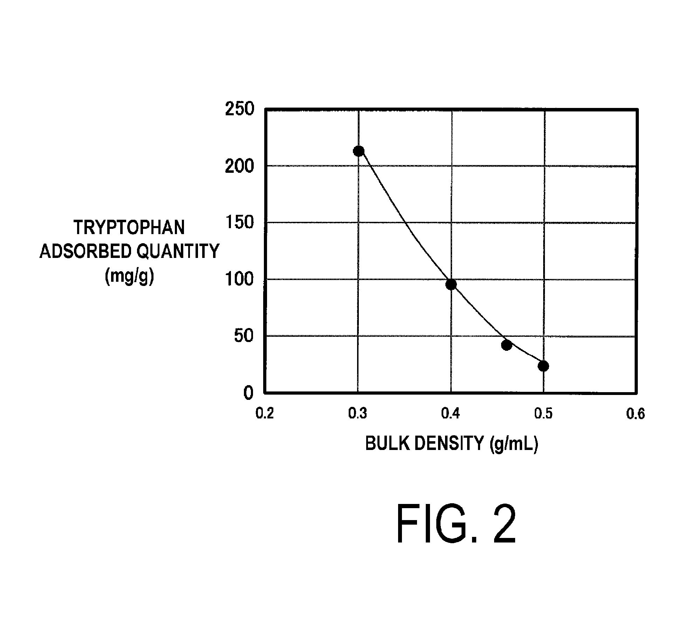

Absorbance at wavelength 278 nm of the sample solution and calibration curve solutions was measured using high-performance liquid chromatography (HPLC), and the tryptophan adsorbed quantity (mg/g) was calculated. The results are shown in Table 2. Furthermore, the results of Working Examples 1 to 3 and Comparative Example 1 are shown in FIG. 2. As is clear from FIG. 2, as bulk density of spherical activated carbon decreases, tryptophan adsorbed quantity increases dramatically in the presence of bile acid.

[Table 1]

| | Carbon source | Average particle size ($\mu$m) | Bulk density (g/mL) | Specific surface area (m²/g) | | Pore volume | | | |
|---|---|---|---|---|---|---|---|---|---|
| | | | | Langmuir method | BET method | $V_{0.64}$ | $V_{1.1}$ | $V_{2.0}$ | $V_m$ |
| Working Example 1 | Crosslinked vinyl resin | 119 | 0.46 | 2820 | 2060 | 0.385 | 0.617 | 0.861 | 1.05 |
| Working Example 2 | Crosslinked vinyl resin | 120 | 0.40 | 3190 | 2350 | 0.391 | 0.646 | 0.968 | 1.26 |
| Working Example 3 | Crosslinked vinyl resin | 78 | 0.30 | 3430 | 2560 | 0.409 | 0.701 | 1.091 | 1.34 |
| Working Example 4 | Phenol resin | 75 | 0.40 | 3290 | 2400 | 0.395 | 0.655 | 0.989 | 1.28 |
| Working Example 5 | Pitch | 91 | 0.38 | 3060 | 2220 | 0.363 | 0.603 | 0.923 | 1.33 |
| Comparative Example 1 | Crosslinked vinyl resin | 127 | 0.50 | 2320 | 1780 | 0.375 | 0.581 | 0.760 | 0.87 |
| Comparative Example 2 | Crosslinked vinyl resin | - | 0.25 | - | - | - | - | - | - |
| Comparative Example 3 | Pitch | - | 0.40 | - | - | - | - | - | - |
| Comparative Example 4 | Crosslinked vinyl resin | 120 | 0.49 | 2440 | 1790 | 0.365 | 0.561 | 0.761 | 1.02 |

(continued)

|  | Carbon source | Average particle size ($\mu$m) | Bulk density (g/mL) | Specific surface area ($m^2$/g) | | Pore volume | | | |
|---|---|---|---|---|---|---|---|---|---|
|  |  |  |  | Langmuir method | BET method | $V_{0.64}$ | $V_{1.1}$ | $V_{2.0}$ | $V_m$ |
| Comparative Example 5 | Phenol resin | 80 | 0.46 | 2540 | 1960 | 0.421 | 0.652 | 0.834 | 0.79 |

[Table 2]

|  |  | Bulk density (g/mL) | Pore volume of pore diameter from 20 nm to 10,000 nm (mL/g) | Pore volume of pore diameter from 7.5 nm to 15,000 nm (mL/g) | Total acidic group content (meq/g) | Adsorbed quantity in presence of sodium cholate (mg/g) | |
|---|---|---|---|---|---|---|---|
|  |  |  |  |  |  | Tryptophan | Potassium indoxyl sulfate |
| Working Example 1 | Crosslinked vinyl resin | 0.46 | 0.05 | 0.14 | 0.14 | 42 | 19 |
| Working Example 2 | Crosslinked vinyl resin | 0.40 | 0.07 | 0.25 | 0.16 | 96 | 31 |
| Working Example 3 | Crosslinked vinyl resin | 0.30 | 0.21 | 0.86 | 0.16 | 213 | 59 |
| Working Example 4 | Phenol resin | 0.40 | 0.08 | 0.29 | 0.27 | 209 | 21 |
| Working Example 5 | Pitch | 0.38 | 0.17 | 0.43 | 0.13 | 99 | 36 |
| Comparative Example 1 | Crosslinked vinyl resin | 0.50 | 0.03 | 0.08 | 0.16 | 24 | 12 |
| Comparative Example 2 | Crosslinked vinyl resin | 0.25 | - | - | - | - | - |
| Comparative Example 3 | Pitch | 0.40 | - | - | - | - | - |
| Comparative Example 4 | Crosslinked vinyl resin | 0.49 | 0.03 | 0.09 | 2.01 | 24 | 13 |
| Comparative Example 5 | Phenol resin | 0.46 | 0.10 | 0.16 | 0.20 | 25 | 15 |

Industrial Applicability

The orally administered adsorbent of the present invention may be used as an orally administered adsorbent for therapy or prophylaxis of a renal disease or may be used as an adsorbent for therapy or prophylaxis of a hepatic disease. Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is pre-dialysis mild renal failure, and it may be used for condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition). Examples of the hepatic disease include fulminant hepatitis, chronic hepatitis, viral hepatitis, alcoholic hepatitis, hepatic fibrosis, cirrhosis, hepatic cancer, autoimmune hepatitis, drug-induced allergic hepatic disorder, primary biliary cirrhosis, tremor, encephalopathy, metabolic disorder, and functional disorder. Otherwise, it may also be used in therapy of illnesses caused by toxic substances present in the body, that is, mental illness and the like.

The present invention has been described above using specific modes of embodiment, but modifications and improvements apparent to persons having ordinary skill in the art are also included in the scope of the present invention.

**Claims**

1. An orally administered adsorbent comprising spherical activated carbon having bulk density from 0.30 g/mL to 0.46 g/mL, a specific surface area determined by the Brunauer-Emmett-Teller method (BET) of not less than 2000 $m^2$/g, total acidic group content of less than 0.30 meq/g, pore volume of pore diameter from 20 nm to 10,000 nm of not greater than 0.21 mL/g, and a micropore volume ratio (Vm) of not less than 1.0, determined by Formula (1): Formula (1)

$$Vm = (V_{2.0} - V_{1.1})/(V_{1.1} - V_{0.64}) \qquad (1)$$

[wherein, in the formula, $V_{2.0}$, $V_{1.1}$, and $V_{0.64}$ are cumulative pore volumes of pore diameter not greater than 2.0 nm, not greater than 1.1 nm, and not greater than 0.64 nm, respectively, calculated by the SF method from nitrogen adsorbed quantity].

2. The orally administered adsorbent according to claim 1, wherein the average particle size of the spherical activated carbon is from 50 $\mu$m to 200 $\mu$m.

3. The orally administered adsorbent according to claim 1 or 2, wherein the spherical activated carbon is prepared using crosslinked vinyl resin as a carbon source.

4. A therapeutic or prophylactic agent for a renal disease containing as an active ingredient the orally administered adsorbent described in any one of claims 1 to 3.

5. A therapeutic or prophylactic agent for a hepatic disease containing as an active ingredient the orally administered adsorbent described in any one of claims 1 to 3.

FIG. 1

FIG. 2

## INTERNATIONAL SEARCH REPORT

| International application No. |
| --- |
| PCT/JP2014/054261 |

### A. CLASSIFICATION OF SUBJECT MATTER

*A61K33/44*(2006.01)i, *A61P1/16*(2006.01)i, *A61P13/12*(2006.01)i, *A61P39/02*(2006.01)i, *B01J20/20*(2006.01)i, *C01B31/10*(2006.01)i

According to International Patent Classification (IPC) or to both national classification and IPC

### B. FIELDS SEARCHED

Minimum documentation searched (classification system followed by classification symbols)
A61K33/44, A61P1/16, A61P13/12, A61P39/02, B01J20/20, C01B31/10

Documentation searched other than minimum documentation to the extent that such documents are included in the fields searched
Jitsuyo Shinan Koho          1922–1996     Jitsuyo Shinan Toroku Koho     1996–2014
Kokai Jitsuyo Shinan Koho     1971–2014     Toroku Jitsuyo Shinan Koho     1994–2014

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2005/094844 A1  (Kureha Chemical Industry Co., Ltd.), 13 October 2005 (13.10.2005), claims 1 to 3; paragraph [0015]; examples 5, 7, 8, 10 & US 2008/0044477 A1     & EP 1745792 A1 | 1–5 |
| Y | JP 2012-102072 A  (Futamura Chemical Co., Ltd.), 31 May 2012 (31.05.2012), paragraph [0028] & US 2013/0202664 A1     & EP 2628483 A1 & WO 2012/050025 A | 1–5 |

☒ Further documents are listed in the continuation of Box C.          ☐ See patent family annex.

| * | Special categories of cited documents: |
| --- | --- |
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 04 April, 2014 (04.04.14) | 15 April, 2014 (15.04.14) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2014/054261

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2010/001485 A1 (Asahi Organic Chemicals Industry Co., Ltd.), 07 January 2010 (07.01.2010), paragraph [0018]; tables 1, 2 (Family: none) | 1-5 |
| A | WO 2008/152994 A1 (Teikoku Medix Co., Ltd.), 18 December 2008 (18.12.2008), claims 1 to 3; paragraph [0033]; table 1 & WO 2008/152994 A1 | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6211611 B
- JP 2006131461 A
- JP 2006015334 A
- JP 2011037749 A

**Non-patent literature cited in the description**

- *Japanese Journal of Nephrology,* 1990, vol. 32 (6), 65-71
- *Japanese Journal of Clinical Dialysis,* 1998, vol. 14 (4), 433-438
- **SAITO, A. ; FOLEY, H.C.** *Ache Journal,* 1991, vol. 37 (3), 429
- **HORVATH, G. ; KAWAZOE, K.** *J. Chem. Eng. Japan,* 1983, vol. 16 (6), 470
- **SAITO, A. ; FOLEY, H.C.** *AIChE Journal,* 1991, vol. 37 (3), 429
- **N. HONDA ; K. KOISO ; K. KUROGAWA.** Clinical Nephrology. Asakura Publishing, 1990
- Nephrology. 1981